# EUROPEAN PATENT SPECIFICATION

(11) **EP 0 668 908 B1**
(45) Date of publication and mention of the grant of the patent: **03.05.2000**
(21) Application number: 91915017.7
(22) Date of filing: 15.08.1991
(51) Int. Cl.: C12N 15/12, C12Q 1/68, G01N 33/50, A61K 39/00

(54) **SPECIFIC DNA SEQUENCES OF A NEMATODE WHICH CAN BE USED FOR THE DIAGNOSIS OF INFECTION WITH THE NEMATODE**
DNS-SEQUENZEN VON EINER NEMATODE, VERWENDBARE FÜR DIAGNOSE VON INFEKTIONEN
SEQUENCES D'ADN SPECIFIQUES D'UN NEMATODE APTES A SERVIR A DIAGNOSTIQUER UNE INFECTION PAR CE NEMATODE

(30) Priority: 16.08.1990 NL 9001832
(43) Date of publication of application: 30.08.1995
(73) Proprietor: Draupnir Corporation N.V., Curaçao (AN); UNIVERSITEIT UTRECHT, 3508 TD Utrecht (NL)
(72) Inventor: ROOS, Maria, Helena, NL-3971 HN Driebergen (NL)
(74) Representative: de Bruijn, Leendert C.
(86) International application number: NL9100153
(87) International publication number: WO9203549

(56) References cited:
- Parasitology Today, volume 6, no. 4, April 1990, M.H. Roos: "The molecular nature of benzimidazole resistance in Helminths", page 125, see page 125, column 1, line 48 - column 2, line 33; page 126, column 3, lines 44-63
- The Journal of Cell Biology, volume 109, no. 6, December 1989, The Rockefeller University Press, M. Driscoll et al.: "Genetic and molecular analysis of a caenorhabditis elegans beta-tubulin that conveys benzimidazole sensitivity", pages 2993-3003, see the whole article
- International Journal for Parasitology, volume 17, no. 3, 1987, Pergamon Journals Ltd (GB), E. Lacey et al.: "Further investigations of the primary mechanism of benzimidazole resistance in haemonchus contortus", pages 1421-1429
- Molecular and Biological Parasitology, volume 43, 4 October 1990, Elsevier Science Publishers B.V., M.H. Roos et al.: "Molecular analysis of selection for benzimidazole resistance in the sheep parasite haemonchus contortus", pages 77-88, see the whole article

## Description

The present invention relates to a method for the diagnosis of a worm infection of nematodes in general and at kits for carrying out said diagnosis, in particular at the diagnosis of Haemonchus contortus.

Furthermore, the invention is also directed at use of specific DNA sequences of a tubulin gene of Haemonchus contortus and parts thereof, at insertions of the sequences in vectors and microorganisms and the use thereof, inter alia for the diagnosis of infection with said nematode.

Parasitic helminths (worms) cause serious infectious diseases in humans and animals. In the case of livestock, infections arise at all ages and in virtually every farm.

The use of anthelmintics is widespread in veterinary medicine. In the Netherlands 35 million guilders were spent on anthelmintics in 1987. In 1985 worldwide spending on anthelmintics was US$ 540 million. The total costs of parasitic worm infections in cattle (anthelmintics, loss of production, etc.) are estimated to be 100 to 200 million guilders per year in the Netherlands (thesis by H.W. Ploeger 1988, Agricultural University of Wageningen).

Little is known about the molecular biology of the mechanism of action of the various control agents. Three groups of broad spectrum anthelmintics are known, each of which have their own mechanism of action. These are
i) benzimidazoles, which probably act via binding to tubulin and consequently inhibit transport within the cell and cell division (Lacey, E., 1988, The role of the cytoskeletal protein, tubulin, in the mode of action and mechanism of drug resistance to benzimidazoles, Int. J. Parasitol. 18, 885-936).
ii) levamisole and morantel, ganglion stimulants which lead to paralysis of the parasite, and
iii) avermectins, which intensify the inhibitory effect of gamma-aminobutyric acid (GABA). In nematodes GABA is the most important neurotransmitter for inhibitory signals from interneurons to motor neurons (Barragry, T., 1984a, Anthelmintics - A review, N.Z. Vet. J. 32, 161-164; Barragry, T., 1984b, Anthelmintics- review Part II, N.Z. Vet. J. 32, 191-199).

Of these three, the benzimidazoles are the most widely used.

Using molecular biological techniques, we have studied one of the most harmful gastrointestinal parasites in sheep: Haemonchus contortus. This occurs worldwide, causes haemonchosis and is responsible for losses of 50 million guilders per year in sheepfarming in the Netherlands.

With a view to effective control of this harmful organism, a sensitive and reliable method for the detection of this worm is indispensable. Parasitic worm infections in sheep are usually mixed infections and a method is therefore needed which is able to differentiate between H. contortus and the other parasitic worms present. The methods which are currently used for the diagnosis of H. contortus are:
1) the detection of the specific symptoms of the disease in sheep, and
2) differentiation of the larval culture from faeces of the sheep.

A disadvantage of 1) lies in the fact that clinical diagnosis of an infection with parasitic intestinal worms is difficult to make because the symptoms of the disease cannot always be differentiated from those of other diseases or poor nutrition. In the case of H. contortus the external symptoms (inter alia anaemia and oedema) are only detectable when there is a severe degree of infection, whereas damage (loss in weight, wool loss, milk loss) is already caused by an infection without these external symptoms of disease.

A disadvantage of method (2) lies in the fact that the eggs of various nematodes in the faeces of infected animals appear to be very similar to one another, as a result of which in the case of eggs it is not possible to make a diagnosis of the worm species. For this purpose, a larval culture must first be carried out, which takes about one week. In practice, this method is little used (Janssens, P.G., Vercruysse, J. and Jansen, J., editors: Wormen en wormziekten bij mens en dier (Worms and worm diseases in humans and animals) (1989) Samsom Stafleu, Alphen aan de Rijn/Brussels).

A new, rapid and species-specific test is therefore needed to be able to diagnose infection with H. contortus. In the case of other nematodes there are a number of tests which are used, such as:
a) the polymerase chain reaction (PCR), and
b) the dot-blot assay.

The dot-blot assay is very sensitive and rapidly yields results, such as, for example, in the diagnosis of the human nematode Brugia, which causes filariasis (McReynolds, L.A., deSimone, S.M., Williams, S.A. (1986) Cloning and comparison of repeated DNA sequences from the human filarial parasite Brugia pahangi, Proc. Natl. Acad. Sci. USA 83, 797-801). In this test, total DNA from the eggs is applied to a nylon membrane and hybridised with the specific DNA probe.

In the case of PCR, specific DNA sequences can be used as primer, by which means a fragment of the specific gene is amplified. This can be rendered visible by analysing the reaction mixture using an agarose gel, or by coupling a (fluorescent) label to the primer (De Bruijn, M.H.L., (1988) Diagnostic DNA amplification: no respite for the elusive parasite, Parasitol. Today, 4:293-295). To date, no Haemmonchus contortus DNA is known which is specific for the species and therefore these tests are not possible for the diagnosis of infection with H. contortus. Surprisingly, it has been found that DNA coding for tubulin is species-specific for H. contortus.

In Parasitology Today volume 6 no 4 April 1990 Roos M.H. et al describe determining whether populations of Haemonchus contortus are of the benzamidazole sensitive type or resistant genotype and suggest using α and β tubulin nucleic acid.

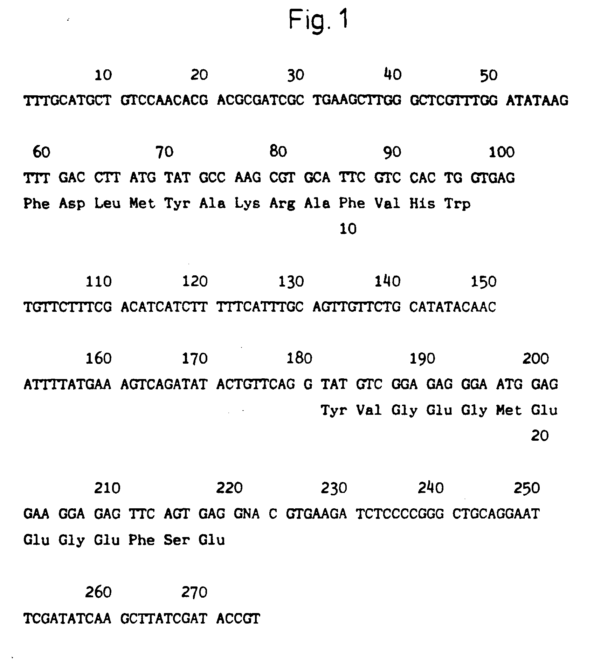

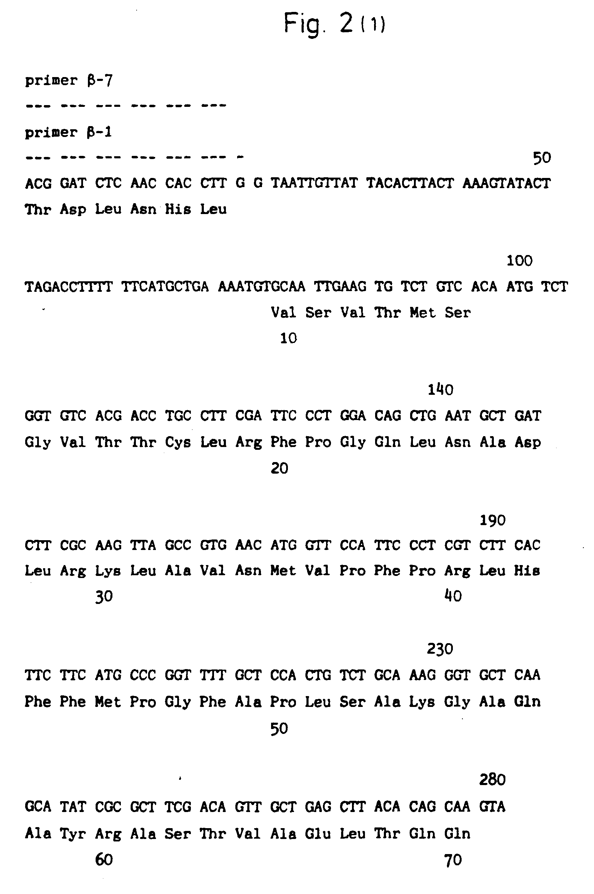

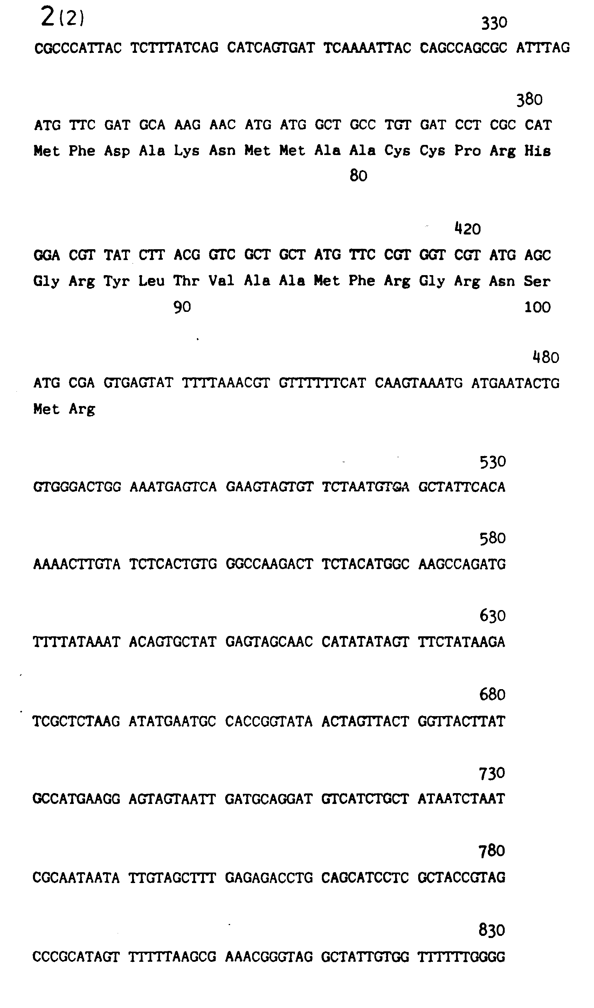

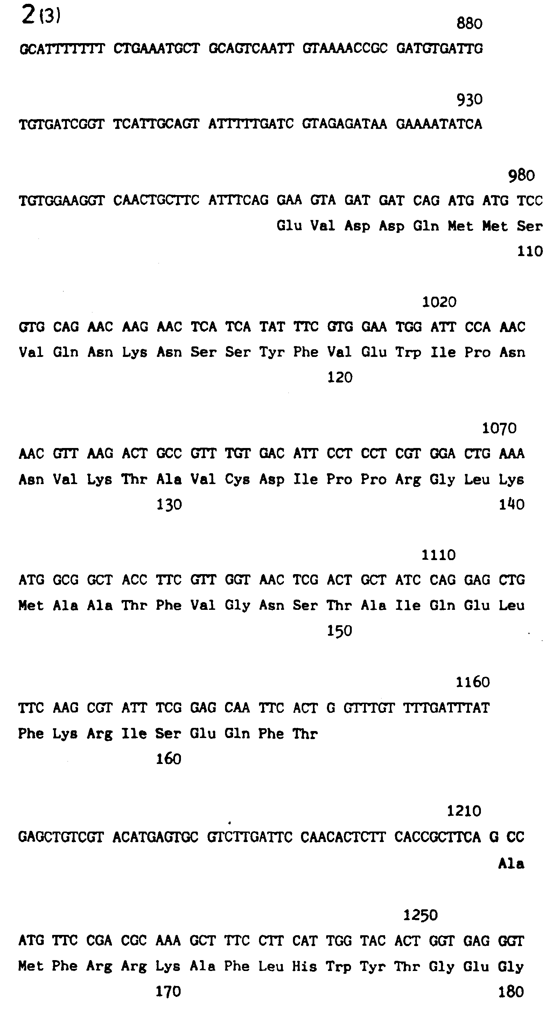

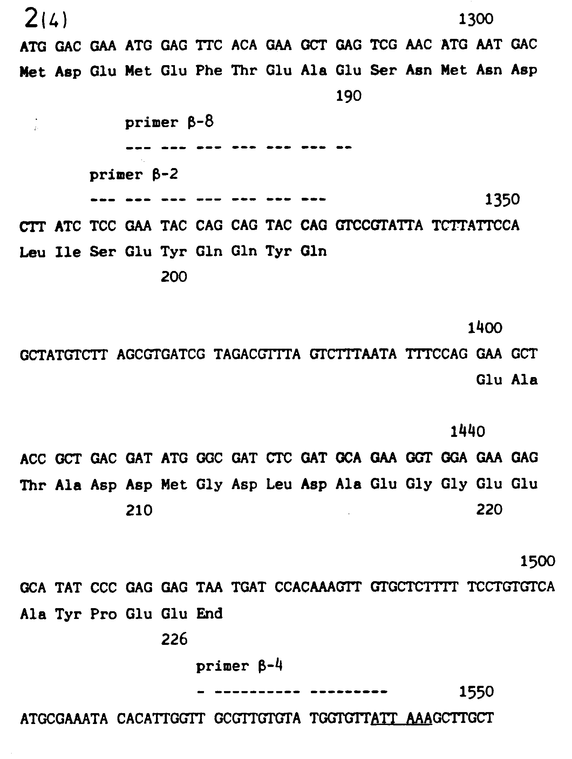

The present invention relates to use of a DNA sequence of a tubulin gene of H. contortus or a part thereof. The DNA sequence of the tubulin gene or a part thereof comprises the alpha-tubulin gene of H. contortus or a part thereof or the beta-tubulin gene of H. contortus or a part thereof.(In figure 1 the DNA and aminoacid sequence of the α-tubulin clone, determined as described further on in the example are given. The numbering of the aminoacids in the α-tubulin gene is from 395-425) (In figure 2 DNA and amino acid sequence of the β-tubulin clone determined as described further on in the example, are illustrated). The numbering of the DNA bases is given above the DNA sequence, that of the amino acids is given below said sequence. The clone contains the 3' half of the gene and the numbering of the amino acids in the β-tubulin gene is 223-448. The polyadenylation signal (bases 1538-1543) is underlined. The site of the oligonucleotide primers is illustrated with a dashed line above the DNA sequence: β-1, bases 1-19 according to the strand illustrated here; β-2 bases 1311-1331 according to the complementary strand).

It was known from study of previously published DNA-sequences of β-tubulin genes of other organisms that β-tubulin genes are strongly conserved [(Cleveland, D.W. and Sullivan, K.F. (1985) Molecular Biology and genetics of tubulin. Ann. Rev. Biochem., 54; 331-365), (Little, M. and Seehaus, T. (1988) Comparative analysis of tubulin sequences. Comp. Biochem. Physiol., 90B; 655-670), (MacRae, T.H. and Langdon, C.M. (1989) Tubulin synthesis, structure and function: what are the relationships? Biochem. Cell Biol., 67; 770-789)].

Furthermore, it was apparent from previously published DNA-sequences of a number of nematodes (also of β-tubulin, see Driscoll et al. The Journal of Biology, 1989, volume 109, pages 2993-3003) that the known nematode DNA sequences mostly resemble higher eucaryotic tubulin sequences. Various β-tubulin genes occur in most higher eucaryotes. From what is presently known there appears to be more difference between the different types (genes) of β-tubulin within one animal species than between the same type among different animal species. For example: the degree of conservation between tubulin type β-4 and β-5 of the human is 91,2 %, whereas conservation between type β-4 of the human and type β-4 of the chicken is 99,3 %.

Considering the state of the art as described above it is therefore surprising that DNA of β-tubulin is species specific for H. contortus.

Fragments of a DNA-sequence according to the invention can be used as oligonucleotide probes for specifically determining H. contortus especially in mixed infections. Preferably the PCR method is used for this. Probes comprising a part of the sequence according to the invention react specifically with, H. contortus, whereby a probe comprising a part of the β-tubulin sequence is especially suited for use in specifically determining H. contortus in this manner.

Upon studying the conservation of DNA of tubulin genes it was found that the location of the introns is generally conserved. Introns generally occur in the 5' half of the tubulin gene. In the article of Driscoll et al. already cited it was demonstrated that introns also occur in the 3' half of the 3 β-tubulin genes of the nematode C. elegans. These introns can be distinguished from the introns of the β-tubulin genes of other higher eucaryotes.

It has now been found that the DNA-sequence of the 3' half of the β-tubulin gene of H. contortus also contains introns. The 3' half of β-tubulin of H. contortus has been found to contain a lot more introns than the 3' half of β-tubulin of C. elegans and the introns also differ in length from the introns of C. elegans. The location and length of introns in the 3' half of the β-tubulin gene appears not to be conserved in nematodes.

Further research of β-tubulin genes of other nematodes further to the above mentioned discovery have revealed that there are other nematodes beside H. contortus and C. elegans which also contain non conserved introns in the 3' half of their β-tubulin genes.

The subject invention is therefore in general directed at the use of a DNA sequence of a specific nematode as oligonucleotide probe, which probe comprises a part of the DNA sequence of the 3' half of a β-tubulin gene, for specifically determining the specific nematode. The subject invention is therefore not restricted to a method for specifically detecting H. contortus in which method a DNA sequence is used as oligonucleotide probe comprising a part of the 3' half of the β-tubulin gene of H. contortus. The detection of H. contortus in this manner serves as an example of a method for the diagnosis of an infection with a certain nematode according to the subject invention.

Another suitable example of a nematode which can specifically be detected using an oligonucleotide probe comprising a part of the DNA sequence of the 3' half of the β-tubulin gene is Trichostrongylus colubriformis.

The presence of differences in the 3' half of β-tubulin genes of nematodes when compared with eachother made a species specific PCR possible. Parts of a DNA sequence comprising a β-tubulin gene can be used as oligonucleotide probes for specifically detecting a nematode with the aid of the PCR in mixed infections.

A particular embodiment of the method that has been described above comprises the use of parts of the β-tubulin gene as primers, which primers are conserved in various nematodes, whereby two sequences are selected as primers which are located in such a manner that the DNA sequence which is amplified from the total DNA of the nematode via PCR, the amplification product, has a species specific length per nematode. because the DNA sequence which is located between the primers is species specific. It is preferable to amplify DNA from the DNA sequence comprising a part of the 3' half of the β-tubulin gene, as the DNA sequence of the 3' half has been found to differ in intron length per nematode.

Two β-tubulin oligonucleotides, β-1 and β-2 were made on the basis of a DNA sequence of the β-tubulin gene of H. contortus as determined by the inventors and illustrated in figure 2, in order to be able to measure the difference in introns among various nematode species. The oligonucleotides namely contain DNA sequences coding for amino acid sequences supposed to be conserved in β-tubulin genes of all higher eucaryotes. Oligonucleotide β-1 comprises amino acids 1-7 of the β-tubulin gene of H. contortus and oligonucleotide β-2 comprises amino acids 198-204. The DNA sequence of β-1 is: 5' ACGGATCTCAACCACCTTG 3' and the sequence of β-2 on the complementary strand is 5' CTGGTACTGCTGGTATTCGGA 3'.

The PCR carried out with these oligonucleotides always gave a positive reaction to the presence of H. contortus DNA also in mixed infections. With this PCR a DNA fragment of approximately 1300 bases was generated. This PCR always gave a positive result when H. contortus DNA was present and then the fragment of 1300 bases was found with the β-1 and β-2 primers. The PCR always gave a negative reaction when no fragment of 1300 bases was present and no H. contortus DNA was present.

The PCR with the β-1 and β-2 primers was also tested on DNA of other prevalent sheep parasites. A positive reaction was also found with Trychostrongylus colubriformis also in mixed infections, however the DNA fragment was smaller approximately 900 bases and clearly discernable from the H. contortus fragment.

The PCR with the β-1 and β-2 primers was also tested on DNA of Ostertagia circumcincta, Nippostrongylus brasiliensis, Trichinella spiralis and Trichinella native. No reaction was found not even with so called degenerate primers, which take into account the degenaracy of the genetic code. These were, primers β-7 (albino acids 1-6) with DNA sequence: 5' (CTA)G(GC)(GTAC)GA(CT)(CT)T(GCAT)AA(CT)CA(CT)(CT)T 3' and β-8 (amino acids 199-205) on the complementary strand with DNA sequence: 5' TC(CT)TGGTA(TC)TG(CT)TG(GA)TA(CT)TC 3' (figure 2). The PCR with the β-7 and β-8 primers was positive with DNA of H. contortus and T. colubriformis giving the DNA fragments that were described previously.

As the used primers correspond to a DNA sequence coding for a very conserved region of the β-tubulin molecule it appears probable that the β-tubulin gene(s) of the nematode species leading to a negative result with these primers using PCR contain an intron in the sequence of the primers. This could prevent the primer from binding and could prevent any reaction taking place. A change in the amino acid sequence in the region of the primers could also be a possible explanation for the above mentioned result but does not seem probable considering the conservation of the amino acid sequence in this region.

By selecting the primers in a different region of the 3' half of the β-tubulin gene a species specific PCR can also be obtained for the nematodes giving a negative result with the primers β-1, β-2, β-7 and β-8.

The subject invention is therefore also directed at a method for the diagnosis of a worm infection, with which method the total DNA from the eggs from the nematode is amplified preferably via PCR, which method is characterized in the use of two parts of the coding sequence of the β-tubulin gene which are conserved in various nematodes as primers, which primers are located in the DNA sequence of the β-tubulin gene in such a manner that the amplification product has a species specific length per nematode.

The beta-tubulin probes comprising the complete 2,2 kb β-tubulin clone of H. contortus (pEMBLaB, deposited at the CBS in Baarn under Nr. CBS 335.90) or the 1,3 kb β-1/β-2 PCR fragment always show a positive reaction to the presence of H. contortus, even in the case of a mixed infection. The beta-tubulin probes of H. contortus were also tested on the DNA of other frequently occurring sheep parasites and this reaction was found to be much less pronounced than with its own species. Yet specific bands were obtained with these large probes which indicates conservation. The weaker reaction could be caused by introns in conserved regions or small species specific DNA sequences within these large probes. The species which were tested are, inter alia: Cooperia circumcincta, Nematodirus battus, Nippostrongylus brasiliensis, Ostertagia circumcincta, Trichostrongylus colubriformis, Trichostrongylus vitrinus, Trichinella spiralis and Trichinella native.

By selecting a specific sequence from a DNA sequence according to the invention of for example, at least 10 nucleotides from a H. contortus specific intron or the DNA sequence coding for amino acids 332-335, which under specific conditions reacts solely with H. contortus, it is possible to establish a test by means of which it is possible, in the case of a mixed infection with nematodes, to diagnose infection with H. contortus from the DNA of the eggs.

Examples of tests which can be used are those which are used in the case of other nematodes:
a) the polymerase chain reaction (PCR), and
b) the dot-blot assay.

The present invention also relates to a method for the diagnosis of a worm infection, in which method the total DNA from the eggs from the nematode is applied to a nylon membrane and hybridised with a DNA probe, which method is characterised in that a diagnosis of infection with Haemonchus contortus is made, using a sequence which corresponds to a specific part of a tubulin gene of H. contortus as oligonucleotide probe. A preferred method is a method in which specific DNA fragments from the DNA of the eggs are amplified, for example by means of PCR, and are rendered visible. The diagnosis of an infection with H. contortus can be made because the amplification of specific fragments is successful when H. contortus DNA is present in the egg sample and sequences are used, such as a primer which corresponds to at least a conserved part of a tubulin gene of H. contortus, whereby said sequences are localised in the DNA sequence of the β-tubulin gene in such a manner that the amplification product has a species specific length.

The invention is also directed at a kit for carrying out the diagnosis of a worm infection which kit comprises at least a species specific part of the 3' half of the β-tubulin gene of the nematode to be detected as oligonucleotide probe and is also provided with other means and materials which are apparent to an expert.

The invention is further directed at a kit for carrying out a method for the diagnosis of infection with H. contortus, which kit comprises at least a part of the DNA sequence according to the invention and is also provided with other means and materials apparent to an expert. Such a kit shall preferably comprise a part of the 3' half of the β-tubulin gene of H. contortus.

The invention is further directed at a kit for carrying a method for the diagnosis of infection with H. contortus, which kit comprises at least primers comprising coding DNA sequences of the β-tubulin gene which are conserved in various nematodes, which primers are located in such a manner in the DNA sequence of the β-tubulin gene of H. contortus that the amplification product has a species specific length. Such a kit shall preferably comprise primers giving a part of the 3' half of the β-tubulin gene of H. contortus as amplification product. Such a kit will comprise for example the β-1 and β-2 primers and/or the β-7 and β-8 primers. Furthermore such a kit is also provided with the means and materials apparent for an expert.

The sequences according to the invention can also be used if they are incorporated in a vector, an expression vector being preferred. The invention relates to vectors of this type and also to recombinant microorganisms which comprise a DNA sequence according to the invention.

The invention also relates to a peptide which comprises at least one fragment of the amino acid sequence which is coded by a DNA sequence according to the invention.

### EXAMPLE

### Construction of the gene libraries of H. contortus and analysis of the tubulin clones

Genomic DNA was made from L3 larvae using the method of Coulson et al. (Coulson, A., Sulston, J., Brenner, S. and Karn, J. (1986) Toward a physical map of the genome of the nematode Caenorhabditis elegans, Proc. Natl. Acad. Sci. USA 83, 7821-7825). The size of the DNA fragments to be cloned was 20 kb. In order to obtain arbitrary fragments of this size a partial digestion was carried out with a restriction enzyme that cuts frequently in genomic DNA. To this end, the concentration of the enzyme was chosen as described in Maniatis et al. (Maniatis, T., Fritsch, T. and Sambrook, J. (1982) Molecular cloning. A laboratory manual, Cold Spring Harbor Laboratory, Cold Spring Harbor, N.Y., USA). These fragments were isolated and ligated in the DNA of the bacteriophage arms of EMBL3 using the method of Frischauf et al. (Frischauf, A.M., Lehrach, H., Poustka, A., Murray, N. (1983) Lambda replacement vectors carrying polylinker sequences, J. Mol. Biol. 170, 827-841). This DNA construct was then packaged in bacteriophage protein envelope and used to infect the bacterial strain Le 392. The infected bacteria were plated out with non-infected bacteria and after incubation at 37°C a plaque was visible at the location where infected bacteria were present (Frischauf, A.M., Lehrach, H., Poustka, A. and Murray, N. (1983) Lambda replacement vectors carrying polylinker sequences, J. Mol. Biol. 170, 827-841). The plaques were then screened according to the method of Maniatis et al. (Maniatis, T., Fritsch, T. and Sambrook, J. (1982) Molecular cloning. A laboratory manual, Cold Spring Harbor Laboratory, Cold Spring Harbor, N.Y., USA). The tubulin probes used to isolate the alpha-tubulin DNA-containing clones were chicken alpha-tubulin [as described in Valenzuela et al. (Valenzuela, P., Quiroga, M., Zaldivar, J., Rutter, W.J., Kirshner, M.W. and Cleveland, D.W. (1981) Nucleotide and corresponding amino acid sequences encoded by alpha and beta tubulin mRNAs. Nature 289, 650-655)] and Drosophila hydei alpha-tubulin [a gift from Dr. R. Brand of the Catholic University of Nijmegen (Michiels, F., Falkenburg. D., Muller, A.M., Hinz, U., Otto, U., Bellmann, R., Glazer, K.H., Brand, R., Bialojan, S. and Renkawitz-Pohl, R. (1987) Testis-specific beta2 tubulins are identical in Drosophila melanogaster and D. hydei but differ from the ubiquitous beta1 tubulin)].

The beta-tubulin probes used to isolate the beta-tubulin DNA-containing clones were a beta-tubulin clone of Caenorhabditis elegans, Ben-1 [which is described in Driscoll et al. (Driscoll, M., Dean, E., Reilly, E., Bergholz, E. and Chalfie, M. (1989) Genetic and molecular analysis of a Caenorhabditis elegans beta tubulin that conveys benzimidazole sensitivity, J. Cell Biol. 109, 2993-3003)] and a beta-tubulin clone of Drosophila hydei pTB5 [a gift from Dr. R. Brand of the Catholic University of Nijmegen (Michiels. F., Falkenburg, D., Muller, A.M., Hinz, U., Otto, U., Bellmann, R., Glazer, K.H., Brand, R., Bialojan, S. and Renkawitz-Pohl, R. (1987) Testis-specific beta2 tubulins are identical in Drosophila melanogaster and D. hydei but differ from the ubiquitous beta1 tubulin)].

Of the clones obtained in this way, the clone pEMBL8cA (deposited with the CBS in Baarn under No. CBS 335.90), for alpha-tubulin, and the clone pEMBL7aB (deposited with the CBS in Baarn under No. CBS 334.90), for beta-tubulin, were further characterised. Subclones thereof, which contain DNA fragments coding for protein, were constructed in the vectors Bluescript and M13. The DNA of these subclones was sequenced using the dideoxy method of Sanger et al. (Sanger, F., Nicklen, S. and Coulson, A.R. (1977) DNA sequencing with chain terminating inhibitors, Proc. Natl. Acad. Sci. USA 74, 5463-5467), as modified in the T7 sequencing kit from Pharmacia and the M13 sequencing kit from Amersham. Standard primers, which are supplied with the kits, were used. The analysis and comparison of the sequences thus obtained was carried out using the Microgenie (Beckman) and PC/Gene (Genofit, Geneva) computer programs. The sequences determined for these clones are shown in Figures 1 and 2 respectively.

### The PCR specific for H. contortus and T. colubriformis.

The oligonucleotides β-1 and β-2 as described above were made in order to carry out a species specific PCR for DNA of H. contortus and T. colubriformis. The β-7 and β-8 primers were made in order to obtain a species specific PCR for the species O. circumcincta, T. spiralis and T. nativa. The PCR method was used according to Ehrlich et al. (Saiki, R.K., (1989) in: PCR technology, principles and applications for DNA amplification, Erlich, H.A. (ed.), Stockton Press, New York, London, pp. 7-16). The reaction conditions used in the present invention, which were used to amplify the DNA, are: PCR with β-1 and β-2 primers with a magnesium concentration of 1,5 mM an annealing temperature of 61°C for 30 seconds, elongation at 72°C for 2 minutes and denaturing at 95°C for 30 seconds. This method amplifies the beta-tubulin of H. contortus and T. colubriformis from the genomic DNA, which can be rendered visible by electrophoresis in an agarose gel, whereby DNA of H. contortus gives a visible band of 1,3 kb and DNA of T. colubriformis gives a visible band of 0,9 kb. This method does not work with Ostertagia, N, brasiliensis, T. spiralis and T. nativa, which makes it clear that this PCR method with the abovementioned primers is specific for Haemonchus contortus and Trichostrongylus colubriformis.

The PCR conditions with the β-7 and β-8 primers were: 5 cycli as follows. An annealing temperature of 37°C for 1 minute, a slow rise in 5 minutes from 37°C to the elongation temperature of 72°C, 2 minutes at 72°C, 1 minute at 95°C. Subsequently 20 cycli of 1 minute at 55°C, 2 minutes at 72°C and 1 minute at 95°C. The magnesium concentration was 1,5 mM. This PCR visualized a positive reaction with H. contortus DNA and was negative with O. circumcincta, T. spiralis and T. nativa.

## Claims

1. A method for differentiated diagnosis of nematode infection with a specific type of nematode useful in the case of mixed nematode infections said method comprising use of a DNA sequence, said DNA sequence being the β-tubulin gene or a part of said gene of said nematode as oligonucleotide probe and/or primer, said part comprising at least 10, preferably at least 18 nucleotides.

2. A method according to claim 1 wherein the specific nematode is Haemonchus contortus and the β-tubulin gene is the β-tubulin gene of Haemonchus contortus.

3. A method according to claim 1 or 2 wherein the probe and/or primer comprises a part of the 3' half of the β-tubulin gene.

4. A method according to any of claims 1-3 wherein the probe and/or primer is species specific.

5. A method according to claim 4, wherein the probe and/or primer is at least 10 nucleotides from a H. contortus specific intron.

6. A method according to any of claims 1-4 wherein the probe is derived from the DNA sequence encoding amino acids 332-335 of H. contortus β-tubulin.

7. A method according to any of claims 1-6 wherein the total nematode DNA is applied to a nylon membrane and hybridised with the probe.

8. A method according to any of claims 1-3 wherein a primer is selected that is conserved in various nematode types, whereby the primer is located such that the DNA that is amplified from the total DNA of the nematode has a species specific length per nematode.

9. A method according to claim 8 wherein the primer is a coding sequence for a conserved amino acid sequence.

10. A method acording to claim 9 wherein two primers are selected that are conserved in various nematodes, which primers are located in the β-tubulin sequence such that the amplification product has species specific length per nematode.

11. A method according to any of claims 1-3 or 8-10, wherein any amplification product of the nematode DNA comprises a part of the 3' half of the β-tubulin gene.

12. A method according to any of claims 1-3 or 8-11, characterised in that β-1 and β-2 are used as primers, whereby β-1 has the sequence 5' ACGGATCTCAACCACCTTG 3' and β-2 has the sequence 5' CTGGTACTGCTGGTATTCGGA 3' on the complementary strand, or primers β-7 and β-8 are used whereby β-7 comprises the amino acids 1-6 of β-tubulin of H. contortus with DNA sequence: 5' (CTA)G(GC)(GTAC)GA(CT)(CT)T(GCAT)AA(CT)CA(CT)(CT)T 3' and β-8 comprises the amino acids 199-205 of β-tubulin of H. contortus with DNA sequence. 5' TC(CT)TGGTA(TC)TG(CT)TG(GA)TA(CT)TC 3' on the complementary strand.

13. A method according to any of the preceding claims wherein H. contortus infection can be differentiated from infection with the various nematodes Cooperia circumcincta., Ostertagia circumcincta, Nematodirus battus, Nippostrongylus brasiliensis, Trichinella spiralis, Trichinella nativa, Trichostrongylus vitrinus and Trichostrongylus colubriformis.

14. A method according to any of claims 1-3 and 8-13, characterised in that a diagnosis of infection is carried out which is specific for H. contortus when the amplification product has a length of 1,3 kb.

15. A method according to any of claims 1-3, 8-14, characterised in that a diagnosis of infection is carried out which is specific for Trichostrongylus colubriformis when the amplification product has a length of 0,9 kb.

16. Kit for carrying out a differentiated diagnosis of infection with Haemonchus contortus according to any of the preceding claims, which kit comprises at least a primer comprising DNA coding sequence, said DNA coding sequence being the 3' half of the β-tubulin gene comprised in the nematode Haemonchus contortus in the plasmid deposited under accession number CBS 334.90 at the Centraal Bureau Schimmelcultures in Baarn The Netherlands, said primer having a length of at least 10 nucleotides preferably of at least 18, said primer being a sequence conserved in various nematodes and located in the DNA sequence of Haemonchus contortus such that the amplification product thereof has a species specific length.

17. Kit according to claim 16 which kit comprises the primers with nucleic acid sequences β-1 and β-2 and/or the primers β-7 and β-8 wherein
β-1 =5'ACGGATCTCAACCACCTTG 3'
β-2 =5'CTGGTACTGCTGGTATTCGGA 3'
β-7 =5'(CTA)G(GC)(GTAC)GA(CT)(CT)T(GCAT)AA(CT)CA(CT)(CT)T 3'
β-8 =5'TC(CT)TGGTA(TC)TG(CT)TG(GA)TA(CT)TC 3'.

18. Kit for carrying out a differentiated diagnosis of infection with Haemonchus contortus according to any of claims 1-15 which kit comprises
at least a probe and/or primer species specific for Haemonchus contortus said probe or primer being derived from a DNA coding sequence, said DNA coding sequence being the 3' half of the β-tubulin gene of Haemonchus contortus comprised in the plasmid deposited under accession number CBS 334.90 at the Centraal Bureau Schimmelcultures in Baarn The Netherlands.

19. Kit according to claim 18 which kit comprises the primers with nucleic acid sequences β-1 and β-2 and/or the primers β-7 and β-8 wherein
β-1 =5'ACGGATCTCAACCACCTTG 3'
β-2 =5'CTGGTACTGCTGGTATTCGGA 3'
β-7 =5'(CTA)G(GC)(GTAC)GA(CT)(CT)T(GCAT)AA(CT)CA(CT)(CT)T 3'
β-8 =5'TC(CT)TGGTA(TC)TG(CT)TG(GA)TA(CT)TC 3'.

## Patentansprüche

1. Verfahren zur differenzierten Diagnose einer NematodenInfektion mit einem speziellen Nematoden-Typ, das im Fall von gemischten Nematoden-Infektionen anwendbar ist, wobei bei dem Verfahren eine DNA-Sequenz, die das β-Tubulin-Gen oder ein Teil dieses Gens dieser Nematode ist, als Oligonukleotid-Sonde und/oder Primer verwendet wird, und der Teil mindestens 10, vorzugsweise mindestens 18 Nukleotide, umfaßt.

2. Verfahren nach Anspruch 1, bei dem die spezielle Nematode Haemonchus contortus und das β-Tubulin-Gen das β-Tubulin-Gen von Haemonchus contortus ist.

3. Verfahren nach Anspruch 1 oder 2, bei dem die Sonde und/oder der Primer einen Teil der 3'-Hälfte des β-Tubulin-Gens umfaßt.

4. Verfahren nach einem der Ansprüche 1-3, bei dem die Sonde und/oder der Primer artenspezifisch ist.

5. Verfahren nach Anspruch 4, bei dem es sich bei der Sonde und/oder dem Primer um mindestens 10 Nukleotide eines für H. contortus spezifischen Introns handelt.

6. Verfahren nach einem der Ansprüche 1-4, bei dem die Sonde von der DNA-Sequenz stammt, welche die Aminosäuren 332-335 des β-Tubulins von H. contortus kodiert.

7. Verfahren nach einem der Ansprüche 1-6, bei dem die gesamte Nematoden-DNA auf eine Nylon-Membran aufgebracht und mit der Sonde hybridisiert wird.

8. Verfahren nach einem der Ansprüche 1-3, bei dem ein Primer ausgewählt wird, der in verschiedenen Nematoden-Typen konserviert ist, wobei der Primer so lokalisiert ist, daß die von der Gesamt-DNA der Nematode amplifizierte DNA für jede Nematode eine artspezifische Länge hat.

9. Verfahren nach Anspruch 8, bei dem der Primer eine kodierende Sequenz für eine konservierte Aminosäuresequenz ist.

10. Verfahren nach Anspruch 9, bei dem zwei Primer ausgewählt werden, die in verschiedenen Nematoden konserviert sind, wobei die Primer in der β-Tubulin-Sequenz so lokalisiert sind, daß das Amplifikationsprodukt jeder Nematode eine artenspezifische Länge hat.

11. Verfahren nach einem der Ansprüche 1-3 oder 8-10, bei dem jedes Amplifikationsprodukt der Nematoden-DNA einen Teil der 3'-Hälfte des β-Tubulin-Gens umfaßt.

12. Verfahren nach einem der Ansprüche 1-3 oder 8-11, dadurch gekennzeichnet, daß β-1 und β-2 als Primer verwendet werden, wobei β-1 die Sequenz 5' ACGGATCTCAACCACCTTG 3' und β-2 die Sequenz 5' CTGGTACTGCTGGTATTCGGA 3' auf dem komplementären Strang hat, oder die Primer β-7 und β-8 verwendet werden, wobei β-7 die Aminosäuren 1-6 des β-Tubulins von H. contortus mit der DNA-Sequenz
5' (CTA)G(GC)(GTAC)GA(CT)(CT)T(GCAT)AA(CT)CA(CT)(CT)T 3'
und β-8 die Aminosäuren 199-205 des β-Tubulins von H. contortus mit der DNA-Sequenz
5' TC(CT)TGGTA(TC)TG(CT)(TG)(GA)TA(CT)TC 3'
auf dem komplementären Strang umfaßt.

13. Verfahren nach einem der vorhergehenden Ansprüche, mit dem die Infektion mit H. contortus von Infektionen mit den unterschiedlichen Nematoden Cooperia circumcincta, Ostertagia circumcincta, Nematodirus battus, Nippostrongylus brasiliensis, Trichinella spiralis, Trichinella nativa, Trichostrongylus vitrinuns und Trichostrongylus colubriformis differenziert werden kann.

14. Verfahren nach einem der Ansprüche 1-3 und 8-13, dadurch gekennzeichnet, daß eine für H. contortus spezifische Diagnose der Infektion durchgeführt wird, wenn das Amplifikationsprodukt eine Länge von 1,3 kb hat.

15. Verfahren nach einem der Ansprüche 1-3, 8-14, dadurch gekennzeichnet, daß eine für Trichostrongylus colubriformis spezifische Diagnose der Infektion durchgeführt wird, wenn das Amplifikationsprodukt eine Länge von 0,9 kb hat.

16. Kit zur Durchführung einer differenzierten Diagnose einer Infektion mit Haemonchus contortus nach einem der vorhergehenden Ansprüche, wobei der Kit mindestens einen Primer enthält, der eine kodierende DNA-Sequenz umfaßt, welche die 3'-Hälfte des β-Tubulin-Gens der Nematode Haemonchus contortus ist, das in dem unter der Zugangsnummer CBS 334.90 beim Centraal Bureau Schimmelcultures in Baarn, Niederlande, hinterlegten Plasmid enthalten ist, und wobei der Primer eine Länge von mindestens 10 Nukleotiden, vorzugsweise mindestens 18, hat und wobei der Primer eine in verschiedenen Nematoden konservierte Sequenz ist und in der DNA-Sequenz von Haemonchus contortus so lokalisiert ist, daß deren Amplifikationsprodukt eine artenspezifische Länge hat.

17. Kit nach Anspruch 16, wobei der Kit die Primer mit den Nukleinsäuresequenzen β-1 und β-2 und/oder die Primer β-7 und
β-8 enthält, wobei
β-1 = 5'ACGGATCTCAACCACCTTG 3'
β-2 = 5'CTGGTACTGCTGGTATTCGGA 3'
β-7 = 5'(CTA)G(GC)(GTAC)GA(CT)(CT)T(GCAT)AA(CT)CA(CT)(CT)T 3'
β-8 = 5'TC(CT)TGGTA(TC)TG(CT)TG(GA)TA(CT)TC 3' ist.

18. Kit zur Durchführung einer differenzierten Diagnose einer Infektion mit Haemonchus contortus nach einem der Ansprüche 1-15, wobei der Kit mindestens eine Sonde und/oder einen Primer enthält, die/der für Haemonchus contortus artenspezifisch ist, und wobei die Sonde oder der Primer von einer kodierenden DNA-Sequenz stammt, welche die 3'-Hälfte des β-Tubulin-Gens von Haemonchus contortus ist, das in dem unter der Zugangsnummer CBS 334.90 beim Centraal Bureau Schimmelcultures in Baarn, Niederlande, hinterlegten Plasmid enthalten ist.

19. Kit nach Anspruch 18, wobei der Kit die Primer mit den Nukleinsäuresequenzen β-1 und β-2 und/oder die Primer β-7 und β-8 enthält, wobei
β-1 = 5'ACGGATCTCAACCACCTTG 3'
β-2 = 5'CTGGTACTGCTGGTATTCGGA 3'
β-7 = 5'(CTA)G(GC)(GTAC)GA(CT)(CT)T(GCAT)AA(CT)CA(CT)(CT)T 3'
β-8 = 5'TC(CT)TGGTA(TC)TG(CT)TG(GA)TA(CT)TC 3' ist.

## Revendications

1. Méthode pour le diagnostic différencié d'une infection à nématode par un type spécifique de nématode, utile dans le cas d'infections mixtes par des nématodes, ladite méthode comprenant l'utilisation d'une séquence d'ADN, ladite séquence d'ADN étant le gène de la β-tubuline, ou une partie dudit gène dudit nématode, en tant que sonde et/ou amorce oligonucléotidique, ladite partie comprenant au moins 10, de préférence au moins 18 nucléotides.

2. Méthode selon la revendication 1, dans laquelle le nématode spécifique est Haemonchus contortus et le gène de la β-tubuline est le gène de la β-tubuline d'Haemonchus contortus.

3. Méthode selon la revendication 1 ou la revendication 2, dans laquelle la sonde et/ou l'amorce comprennent une partie de la moitié 3' du gène de la β-tubuline.

4. Méthode selon l'une quelconque des revendications 1 à 3, dans laquelle la sonde et/ou l'amorce sont spécifiques de l'espèce.

5. Méthode selon la revendication 4, dans laquelle la sonde et/ou l'amorce sont constituées d'au moins 10 nucléotides d'un intron spécifique de H. contortus.

6. Méthode selon l'une quelconque des revendications 1 à 4, dans laquelle la sonde est dérivée de la séquence d'ADN codant les acides aminés 332-335 de la β-tubuline de H. contortus.

7. Méthode selon l'une quelconque des revendications 1 à 6, dans laquelle l'ADN total de nématode est appliqué à une membrane de nylon et hybridé avec la sonde.

8. Méthode selon l'une quelconque des revendications 1 à 3, dans laquelle on choisit une amorce qui est conservée chez divers types de nématodes, l'amorce étant ainsi située de telle sorte que l'ADN qui est amplifié à partir de l'ADN total du nématode ait une longueur spécifique de l'espèce pour chaque nématode.

9. Méthode selon la revendication 8, dans laquelle l'amorce est une séquence codante pour une séquence d'acides aminés conservée.

10. Méthode selon la revendication 9, dans laquelle on choisit deux amorces qui sont conservées chez divers nématodes, ces amorces étant situées dans la séquence de la β-tubuline, de telle sorte que le produit d'amplification ait une longueur spécifique de l'espèce pour chaque nématode.

11. Méthode selon l'une quelconque des revendications 1 à 3 ou 8 à 10, dans laquelle tous les produits d'amplification de l'ADN de nématode comprennent une partie de la moitié 3' du gène de la β-tubuline.

12. Méthode selon l'une quelconque des revendications 1 à 3 ou 8 à 11, caractérisée en ce que l'on utilise comme amorce β-1 et β-2, β-1 ayant ainsi la séquence
5' ACGGATCTCAACCACCTTG 3' et β-2 ayant ainsi la séquence
5' CTGGTACTGCTGGTATTCGGA 3' sur le brin complémentaire, ou bien on utilise les amorces β-7 et β-8, β-7 comprenant ainsi les acides aminés 1-6 de la β-tubuline de H. contortus avec la séquence d'ADN :
5' (CTA)G(GC)(GTAC)GA(CT)(CT)T(GCAT)AA(CT)CA(CT)(CT)T 3' et β-8 comprenant ainsi les acides aminés 199-205 de la β-tubuline de H. contortus avec la séquence d'ADN
5' TC(CT)TGGTA(TC)TG(CT)TG(GA)TA(CT)TC 3' sur le brin complémentaire.

13. Méthode selon l'une quelconque des revendications précédentes, dans laquelle une infection par H. contortus peut être différenciée d'une infection par les divers nématodes Cooperia circumcincta, Ostertagia circumcincta, Nematodirus battus, Nippostrongylus brasiliensis, Trichinella spiralis, Trichinella nativa, Trichostrongylus vitrinus et Trichostrongylus colubriformis.

14. Méthode selon l'une quelconque des revendications 1 à 3 et 8 à 13, caractérisée en ce que l'on effectue un diagnostic d'infection qui est spécifique de H. contortus lorsque le produit d'amplification a une longueur de 1,3 kb.

15. Méthode selon l'une quelconque des revendications 1 à 3 et 8 à 14, caractérisée en ce que l'on effectue un diagnostic d'infection qui est spécifique de Trichostrongylus colubriformis lorsque le produit d'amplification a une longueur de 0,9 kb.

16. Kit pour effectuer un diagnostic différencié d'une infection par Haemonchus contortus selon l'une quelconque des revendications précédentes, ce kit comprenant au moins une amorce comprenant une séquence codante d'ADN, ladite séquence codante d'ADN étant la moitié 3' du gène de la β-tubuline comprise dans le nématode Haemonchus contortus dans le plasmide déposé sous le numéro d'accès CBS 334.90 au Centraal Bureau Schimmelcultures à Baarn, Pays-Bas, ladite amorce ayant une longueur d'au moins 10 nucléotides, de préférence d'au moins 18, ladite amorce étant une séquence conservée chez divers nématodes et située dans la séquence d'ADN d'Haemonchus contortus, et telle que son produit d'amplification a une longueur spécifique de l'espèce.

17. Kit selon le revendication 16, ce kit comprenant les amorces ayant les séquences d'acides nucléiques β-1 et β-2 et/ou les amorces β-7 et β-8, dans lequel
β-1 = 5' ACGGATCTCAACCACCTTG 3'
β-2 = 5' CTGGTACTGCTGGTATTCGGA 3'
β-7 = 5'(CTA)G(GC)(GTAC)GA(CT)(CT)T(GCAT)AA(CT)CA(CT)(CT)T3'
β-8 = 5' TC(CT)TGGTA(TC)TG(CT)TG(GA)TA(CT)TC 3'.

18. Kit pour effectuer un diagnostic différencié d'infection par Haemonchus contortus selon l'une quelconque des revendications 1-15, ce kit comprenant au moins une sonde et/ou une amorce spécifique de l'espèce pour Haemonchus contortus, ladite sonde ou amorce étant dérivée d'une séquence codante d'ADN, ladite séquence codante d'ADN étant la moitié 3' du gène de la β-tubuline d'Haemonchus contortus comprise dans le plasmide déposé sous le numéro d'accès CBS 334.90 au Centraal Bureau Schimmelcultures à Baarn, Pays-Bas.

19. Kit selon la revendication 18, ce kit comprenant les amorces ayant les séquences d'acides nucléiques β-1 et β-2 et/ou les amorces β-7 et β-8, dans lequel
β-1 = 5' ACGGATCTCAACCACCTTG 3'
β-2 = 5' CTGGTACTGCTGGTATTCGGA 3'
β-7 = 5' CTA)G(GC)(GTAC)GA(CT)(CT)T(GCAT)AA(CT)CA(CT)(CT)T3'
β-8 = 5' TC(CT)TGGTA(TC)TG(CT)TG(GA)TA(CT)TC 3'.
